# EUROPEAN PATENT APPLICATION

(11) **EP 4 574 809 A1**
(43) Date of publication of application: **25.06.2025**
(21) Application number: 23854869.7
(22) Date of filing: 10.08.2023
(51) Int. Cl.: C07C 63/04, C07C 63/307, C07C 65/03, C07C 229/60, C07F 1/08, B01J 20/22

(54) **DESULFURIZING AGENT**

(30) Priority: 16.08.2022 JP 2022129485
(71) Applicant: Panasonic Intellectual Property Management Co., Ltd., Kadoma-shi, Osaka 571-0057 (JP)
(72) Inventor: TAGUCHI, Kiyoshi, Kadoma-shi, Osaka 571-0057 (JP); OTA, Tomohiro, Kadoma-shi, Osaka 571-0057 (JP); TAKEDA, Kenyu, Kadoma-shi, Osaka 571-0057 (JP)
(74) Representative: Grünecker Patent- und Rechtsanwälte PartG mbB
(86) International application number: PCT/JP2023/029378
(87) International publication number: WO 2024/038835

(57) **Abstract**

A desulfurizing agent according to the present disclosure is a desulfurizing agent for removing a sulfur compound from a raw material gas containing the sulfur compound, the desulfurizing agent including a metal-organic framework, wherein the metal-organic framework includes a copper ion, trimesic acid, and a benzoic acid compound, and the benzoic acid compound is a compound in which a hydrogen atom at a meta position or a para position with respect to a carboxy group of benzoic acid is substituted with a substituent, and has a higher electron-donating property than benzoic acid.

## Description

### TECHNICAL FIELD

The present disclosure relates to a desulfurizing agent.

### BACKGROUND ART

Patent Literature 1 discloses a technique for bringing a fuel gas (raw material gas) containing a sulfide compound (sulfur compound) into contact with a metal-organic framework composed of a copper ion and trimesic acid, thereby removing the sulfide compound (sulfur compound) from the fuel gas (raw material gas).

### CITATION LIST

### Patent Literature

Patent Literature 1: WO 2017/150019 A1

### SUMMARY OF INVENTION

### Technical Problem

The present disclosure provides a desulfurizing agent that can reduce the amount of the desulfurizing agent to be packed in a desulfurizer to downsize the desulfurizer.

### Solution to Problem

A desulfurizing agent according to the present disclosure is a desulfurizing agent for removing a sulfur compound from a raw material gas containing the sulfur compound, the desulfurizing agent including a metal-organic framework, wherein the metal-organic framework includes a copper ion, trimesic acid, and a benzoic acid compound, and the benzoic acid compound is a compound in which a hydrogen atom at a meta position or a para position with respect to a carboxy group of benzoic acid is substituted with a substituent, and has a higher electron-donating property than benzoic acid.

### Advantageous Effects of Invention

In the desulfurizing agent according to the present disclosure, electrons are donated to the copper ion of the metal-organic framework by the benzoic acid compound, which has a higher electron-donating property than benzoic acid, and the affinity between the sulfur compound and the copper ion is increased, thereby improving a sulfur compound adsorption capacity.

In addition, since the metal-organic framework included in the desulfurizing agent according to the present disclosure has a configuration in which a part of trimesic acid in a metal-organic framework composed of a copper ion and trimesic acid is replaced by a benzoic acid compound, the number of pores having a large pore diameter is larger than that in the metal-organic framework composed of a copper ion and trimesic acid, thereby improving the sulfur compound adsorption capacity.

Therefore, it is possible to provide a desulfurizing agent that can reduce the amount of the desulfurizing agent to be packed in a desulfurizer to downsize the desulfurizer.

### BRIEF DESCRIPTION OF THE DRAWINGS

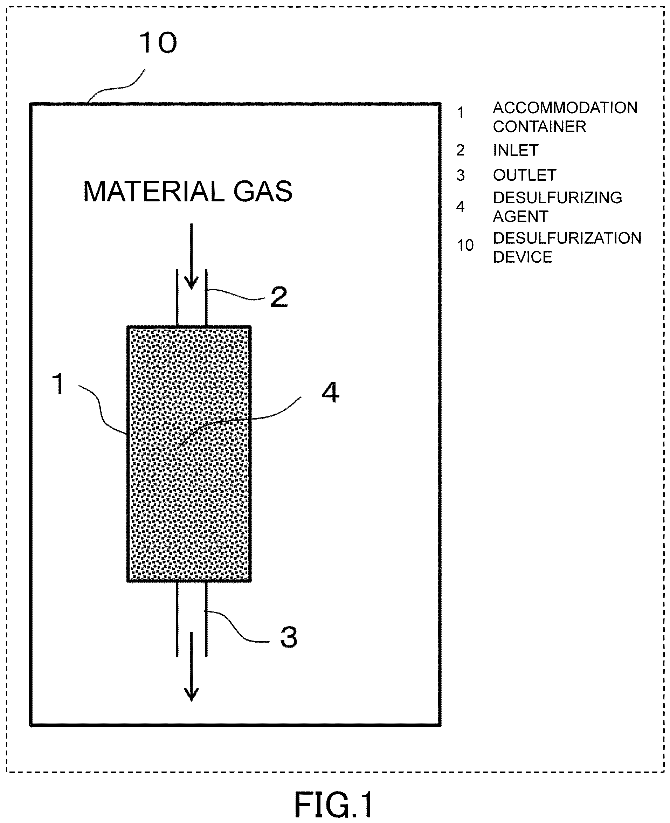

FIG. 1 is a schematic diagram showing a desulfurizer in which a desulfurizing agent according to Embodiment 1 is packed.

### DESCRIPTION OF EMBODIMENTS

### (Findings on which the present disclosure is based, etc.)

At the time when the inventors came to conceive of the present disclosure, there was a metal-organic framework composed of a copper ion and trimesic acid, as a desulfurizing agent for removing a sulfur compound from a raw material gas containing the sulfur compound. Thus, it is possible to simply and effectively remove the sulfur compound contained in the raw material gas.

However, the adsorption amount was low with respect to the theoretical adsorption amount of the sulfur compound expected from the content of the copper ion in the framework serving as an active point, so that it was not possible to downsize a desulfurizer.

Under these circumstances, the inventors obtained an idea that when a metal-organic framework having a configuration in which a part of trimesic acid in a metal-organic framework composed of a copper ion and trimesic acid is replaced by a benzoic acid compound having a higher electron-donating property than benzoic acid is used as a desulfurizing agent, electrons are donated to the copper ion of the metal-organic framework by the benzoic acid compound and the affinity between the sulfur compound and the copper ion is increased, thereby improving the sulfur compound adsorption capacity, and further, the number of pores having a large pore diameter is larger than that in the metal-organic framework composed of a copper ion and trimesic acid, thereby improving the sulfur compound adsorption capacity.

The present disclosure provides a desulfurizing agent that can reduce the volume of the desulfurizing agent to be packed in a desulfurizer to downsize the desulfurizer.

Hereinafter, an embodiment will be described in detail with reference to the drawing. However, unnecessarily more detailed description may be omitted. For example, detailed description of a well-known matter or overlapping description of substantially the same structure may be omitted.

The accompanying drawing and the following description are provided for those skilled in the art to fully understand the present disclosure, and are not intended to limit the subject matter recited in the claims.

### (Embodiment 1)

Hereinafter, Embodiment 1 will be described with reference to FIG. 1.

### [1-1. Configuration]

As shown in FIG. 1, a desulfurizing agent 4 of the present embodiment is packed (loaded) in an accommodation container 1. The accommodation container 1 is provided with an inlet 2, at one end in the longitudinal direction thereof, for allowing a raw material gas before desulfurization to flow into the accommodation container 1, and is provided with an outlet 3, at another end thereof, for allowing the raw material gas after desulfurization to flow out from the inside of the accommodation container 1.

A desulfurization device 10 is configured such that the raw material gas before desulfurization flows into the inlet 2 of the accommodation container 1 and the raw material gas desulfurized by the desulfurizing agent 4 packed (loaded) in the accommodation container 1 flows out from the inside of the accommodation container 1.

The inlet 2 is a pipe through which the raw material gas is supplied.

The raw material gas in the present embodiment is city gas mainly composed of methane, and contains tetrahydrothiophene (hereinafter referred to as THT) at a concentration of 15 ppm as a sulfur compound.

The outlet 3 is a pipe through which the raw material gas from which THT has been removed by the desulfurizing agent 4 is discharged.

The raw material gas discharged from the outlet 3 is supplied to a hydrogen generation apparatus (not shown) that generates hydrogen, is converted to hydrogen by a reforming catalyst (not shown) in the hydrogen generation apparatus, and is used in hydrogen-using equipment (not shown) such as a fuel cell.

The desulfurizing agent 4 includes a metal-organic framework that adsorbs a sulfur compound. The metal-organic framework includes a copper ion, trimesic acid, and a benzoic acid compound. The benzoic acid compound is a compound in which the hydrogen atom at a meta position or a para position with respect to the carboxy group of benzoic acid is substituted with a substituent, and has a higher electron-donating property than benzoic acid.

In the desulfurizing agent 4 in the present embodiment, Cu-BTC-OH-BA-p (Example 1) containing 4-hydroxybenzoic acid having a structure in which the hydrogen atom at a para position with respect to the carboxy group of benzoic acid is substituted with a hydroxy group, was used as the benzoic acid compound.

### (Synthesis method for metal-organic framework)

The metal-organic framework was synthesized by a known solvothermal method.

First, a raw material solution was prepared by adding a copper ion source, trimesic acid, and the benzoic acid compound to an organic solvent. Then, the raw material solution was heated to allow metal-organic framework crystals to grow.

In the obtained metal-organic framework crystals, unreacted copper ion source and trimesic acid remained. Thus, the crystals were washed to remove the unreacted copper ion source and trimesic acid, and then dried to obtain a powdered metal-organic framework.

The molar ratio of the benzoic acid compound included in the metal-organic framework is desirably 10% or more and 50% or less with respect to trimesic acid.

Normally, Cu-BTC has many micropores, but if this molar ratio exceeds 50%, it is thought that the number of mesopores increases due to two or more benzoic acid molecules or isophthalic acid molecules being adjacent to each other. An increase in mesopores means collapse of the three-dimensional structure of the metal-organic framework, and causes a decrease in specific surface area, so that such a metal-organic framework is not suitable for use as a desulfurizing agent. In the present embodiment, the molar ratio of the benzoic acid compound is set to 30% with respect to trimesic acid.

Hereinafter, an example of the synthesis method for Cu-BTC-OH-BA-p which is a metal-organic framework of Example 1 in Embodiment 1 will be described in detail.

In the present embodiment, Cu-BTC-OH-BA-p (Example 1), which was synthesized using 4-hydroxybenzoic acid as the benzoic acid compound, will be described.

First, a raw material solution was prepared by mixing copper sulfate anhydride (3.6 mmol), trimesic acid (1.6 mmol), and 4-hydroxybenzoic acid (0.48 mmol) into ethylene glycol (40 mL). In the raw material solution, the molar ratio of 4-hydroxybenzoic acid to trimesic acid was set to 30%.

The raw material solution was placed in a sealed container and heated at 120°C for 24 hours to obtain a product. The precipitate was further washed and dried at 120°C to obtain Cu-BTC-OH-BA-p (Example 1).

Cu-BTC-CH3-BA-p (Example 2) was synthesized using 4-methylbenzoic acid instead of 4-hydroxybenzoic acid in the metal-organic framework of Example 1. 4-methylbenzoic acid is a benzoic acid compound having a structure in which the hydrogen atom at a para position with respect to the carboxy group of benzoic acid is substituted with a methyl group.

Cu-BTC-NH2-BA-p (Example 3) was synthesized using 4-aminobenzoic acid instead of 4-hydroxybenzoic acid in the metal-organic framework of Example 1. 4-aminobenzoic acid is a benzoic acid compound having a structure in which the hydrogen atom at a para position with respect to the carboxy group of benzoic acid is substituted with an amino group.

Cu-BTC-CH3-BA-m (Example 4) was synthesized using 3-methylbenzoic acid instead of 4-hydroxybenzoic acid in the metal-organic framework of Example 1. 3-methylbenzoic acid is a benzoic acid compound having a structure in which the hydrogen atom at a meta position with respect to the carboxy group of benzoic acid is substituted with a methyl group.

Cu-BTC-NH2-BA-m (Example 5) was synthesized using 3-aminobenzoic acid instead of 4-hydroxybenzoic acid in the metal-organic framework of Example 1. 3-aminobenzoic acid is a benzoic acid compound having a structure in which the hydrogen atom at a meta position with respect to the carboxy group of benzoic acid is substituted with an amino group.

Cu-BTC-BA (Comparative Example 1) was synthesized using benzoic acid instead of 4-hydroxybenzoic acid in the metal-organic framework of Example 1.

Cu-BTC-OH-BA-m (Comparative Example 2) was synthesized using 3-hydroxybenzoic acid instead of 4-hydroxybenzoic acid in the metal-organic framework of Example 1. 3-hydroxybenzoic acid is a benzoic acid compound having a structure in which the hydrogen atom at a meta position with respect to the carboxy group of benzoic acid is substituted with a hydroxy group.

Cu-BTC-NO2-BA-p (Comparative Example 3) was synthesized using 4-nitrobenzoic acid instead of 4-hydroxybenzoic acid in the metal-organic framework of Example 1. 4-nitrobenzoic acid is a benzoic acid compound having a structure in which the hydrogen atom at a para position with respect to the carboxy group of benzoic acid is substituted with a nitro group.

Cu-BTC-Cl-BA-p (Comparative Example 4) was synthesized using 4-chlorobenzoic acid instead of 4-hydroxybenzoic acid in the metal-organic framework of Example 1. 4-chlorobenzoic acid is a benzoic acid compound having a structure in which the hydrogen atom at a para position with respect to the carboxy group of benzoic acid is substituted with a chloro group.

### [1-2. Operation]

The operation and effects of the desulfurizing agent 4 configured as described above will be described below.

A raw material gas containing 15 ppm of THT is supplied to the inlet 2 of the accommodation container 1. The supplied raw material gas passes through the accommodation container 1 while coming into contact with the desulfurizing agent 4 inside the accommodation container 1. The THT is removed from the raw material gas by the desulfurizing agent 4, so that the raw material gas from which the THT has been removed to 20 ppb or less is discharged from the outlet 3.

### (Evaluation of sulfur adsorption performance)

A method for evaluating the sulfur adsorption performance of the desulfurizing agent 4 loaded in the desulfurization device 10 will be described. The sulfur adsorption performance is indicated as a THT adsorption capacity (wt%) which is the ratio of the weight of the sulfur atoms in the adsorbed THT to the weight of the desulfurizing agent 4.

The THT adsorption capacity was measured for multiple equilibrium concentrations by introducing a sample and THT gas into a sealed bag, leaving the sealed bag at 60°C for 24 hours, and then analyzing the THT concentration in the bag, an adsorption isotherm was calculated using Langmuir's equation assuming that adsorptive molecules were adsorbed in a monomolecular layer, and the adsorption capacity at 15 ppm was calculated, thereby evaluating the THT adsorption capacity.

Table 1 shows the THT adsorption capacity at 15 ppm for each of the samples of Examples 1 to 5 and Comparative Examples 1 to 5. In addition, for the samples to which the benzoic acid compounds were added, a σ value (substituent constant determined by the type and position of the substituent among the constants of proportionality in the Hammett rule) indicating the electron-withdrawing property of the benzoic acid compound is also shown. A sample having a negative σ value has a higher electron-donating property than benzoic acid.

**[Table 1]**

| Sample | σ value indicating electron-withdrawing property of benzoic acid compound | THT adsorption capacity at 15 ppm (wt%) |
|---|---|---|
| Example 1: Cu-BTC-OH-BA-p | -0.320 | 13.8 |
| Example 2: Cu-BTC-CH3-BA-p | -0.170 | 13.9 |
| Example 3: Cu-BTC-NH2-BA-p | -0.660 | 14.5 |
| Example 4: Cu-BTC-CH3-BA-m | -0.069 | 11.0 |
| Example 5: Cu-BTC-NH2-BA-m | -0.161 | 12.0 |
| Comparative Example 1: Cu-BTC-BA | 0 | 10.2 |
| Comparative Example 2: Cu-BTC-OH-BA-m | 0.121 | 10.0 |
| Comparative Example 3: Cu-BTC-NO2-BA-p | 0.778 | 5.7 |
| Comparative Example 4: Cu-BTC-Cl-BA-p | 0.227 | 9.4 |
| Comparative Example 5: Cu-BTC | - | 7.4 |

As shown in Table 1, the samples of Examples 1 to 5 having negative σ values showed a higher THT adsorption capacity than Cu-BTC-BA of Comparative Example 1.

In addition, the samples of Comparative Examples 2 to 4 having positive σ values showed a lower THT adsorption capacity than Cu-BTC-BA of Comparative Example 1.

In addition, the metal-organic framework Cu-BTC, which constitutes a metal-organic framework with a copper ion and trimesic acid and includes no benzoic acid compound, had an even lower THT adsorption capacity than Comparative Example 1, as shown in Comparative Example 5.

### [1-3. Effects, etc.]

As described above, in the present embodiment, the desulfurizing agent 4 is a desulfurizing agent for removing a sulfur compound from a raw material gas containing the sulfur compound, the desulfurizing agent 4 includes a metal-organic framework, and the metal-organic framework includes a copper ion, trimesic acid, and a benzoic acid compound. The benzoic acid compound is a compound in which the hydrogen atom at a meta position or a para position with respect to the carboxy group of benzoic acid is substituted with a substituent, and has a higher electron-donating property than benzoic acid.

Accordingly, electrons are donated to the copper ion of the metal-organic framework by the benzoic acid compound, which has a higher electron-donating property than benzoic acid, and the affinity between the sulfur compound and the copper ion is increased, thereby improving the sulfur compound adsorption capacity.

In addition, since the metal-organic framework included in the desulfurizing agent according to the present disclosure has a configuration in which a part of trimesic acid in a metal-organic framework composed of a copper ion and trimesic acid is replaced by a benzoic acid compound, the number of pores having a large pore diameter is larger than that in the metal-organic framework composed of a copper ion and trimesic acid, thereby improving the sulfur compound adsorption capacity.

Therefore, it is possible to provide a desulfurizing agent that can reduce the amount of the desulfurizing agent 4 to be packed in a desulfurizer (accommodation container 1) to downsize the desulfurizer (accommodation container 1).

In addition, the benzoic acid compound may be 4-hydroxybenzoic acid.

Accordingly, electrons are donated to the copper ion of the metal-organic framework by 4-hydroxybenzoic acid, which has a higher electron-donating property than benzoic acid, the affinity between the sulfur compound and the copper ion is increased, and the pores of the metal-organic framework are not blocked due to the small size of the functional group (substituent), thereby further improving the sulfur compound adsorption capacity.

In addition, the benzoic acid compound may be 4-methylbenzoic acid.

Accordingly, electrons are donated to the copper ion of the metal-organic framework by 4-methylbenzoic acid, which has a higher electron-donating property than benzoic acid, and the affinity between the sulfur compound and the copper ion is increased, thereby further improving the sulfur compound adsorption capacity, and it is also possible to reduce the cost of the desulfurizing agent by using 4-methylbenzoic acid which is inexpensive.

Therefore, it is possible to provide a desulfurizing agent that can reduce the amount of the desulfurizing agent 4 to be packed in a desulfurizer (accommodation container 1) to downsize the desulfurizer (accommodation container 1) and that is inexpensive.

In addition, the benzoic acid compound may be 4-aminobenzoic acid.

Accordingly, electrons are donated to the copper ion of the metal-organic framework by 4-aminobenzoic acid, which has a higher electron-donating property than benzoic acid, and the affinity between the sulfur compound and the copper ion is increased. In addition, the affinity with the sulfur compound is further increased by the basic functional group, thereby further improving the sulfur compound adsorption capacity.

Therefore, it is possible to provide a desulfurizing agent that can reduce the amount of the desulfurizing agent 4 to be packed in a desulfurizer (accommodation container 1) to downsize the desulfurizer (accommodation container 1).

### (Other embodiments)

As described above, Embodiment 1 has been described as an illustration of the technique disclosed in the present application. However, the technique according to the present disclosure is not limited to these, and can also be applied to embodiments obtained by making modifications, replacements, additions, omissions, and the like. Furthermore, the components described in Embodiment 1 above can also be combined to obtain a new embodiment.

Thus, other embodiments will be described below.

In Embodiment 1, the city gas, which is mainly composed of methane, is exemplified as the raw material gas, but the raw material gas may also be LPG, natural gas, or hydrogen.

The above embodiments are for exemplification of the technique according to the present disclosure, and accordingly can be subjected to various modifications, replacements, additions, omissions, and the like within the scope of the claims or equivalents thereof.

### INDUSTRIAL APPLICABILITY

The present disclosure is applicable to a desulfurization method for removing a sulfur compound contained in a raw material gas. Specifically, the present disclosure is applicable to a fuel cell system or hydrogen production apparatus equipped with a hydrogen generation apparatus that generates hydrogen from city gas or LPG, a fuel cell system that generates electricity using hydrogen containing a sulfur compound, etc.

## Claims

1. A desulfurizing agent for removing a sulfur compound from a raw material gas containing the sulfur compound, the desulfurizing agent comprising
a metal-organic framework, wherein
the metal-organic framework comprises a copper ion, trimesic acid, and a benzoic acid compound, and
the benzoic acid compound is a compound in which a hydrogen atom at a meta position or a para position with respect to a carboxy group of benzoic acid is substituted with a substituent, and has a higher electron-donating property than benzoic acid.

2. The desulfurizing agent according to claim 1, wherein the benzoic acid compound is 4-hydroxybenzoic acid.

3. The desulfurizing agent according to claim 1, wherein the benzoic acid compound is 4-methylbenzoic acid.

4. The desulfurizing agent according to claim 1, wherein the benzoic acid compound is 4-aminobenzoic acid.
